(19)

**Europäisches Patentamt**
**European Patent Office**
**Office européen des brevets**

(11) **EP 4 640 829 A1**

(12) **EUROPEAN PATENT APPLICATION**
published in accordance with Art. 153(4) EPC

(43) Date of publication:
**29.10.2025 Bulletin 2025/44**

(21) Application number: **23905793.8**

(22) Date of filing: **14.12.2023**

(51) International Patent Classification (IPC):
*C12N 9/20* (2006.01)     *C12N 15/55* (2006.01)
*C12N 15/63* (2006.01)     *C12P 7/649* (2022.01)

(52) Cooperative Patent Classification (CPC):
**C12N 9/14; C12N 9/20; C12N 15/63; C12N 15/81;
C12P 7/649;** C12R 2001/84; Y02E 50/10

(86) International application number:
**PCT/CN2023/138703**

(87) International publication number:
**WO 2024/131628 (27.06.2024 Gazette 2024/26)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **21.12.2022 CN 202211647293**

(71) Applicant: **Wilmar (shanghai) Biotechnology
Research &
Development Center Co., Ltd.
Shanghai 200137 (CN)**

(72) Inventors:
• **XUAN, Yaoji
Shanghai 200137 (CN)**

• **LIU, Guorui
Shanghai 200137 (CN)**
• **WANG, Shengnan
Shanghai 200137 (CN)**
• **DAI, Xiaojun
Shanghai 200137 (CN)**

(74) Representative: **Kaldewey, Tim Merlin et al
Becker & Müller
Patentanwälte
Turmstraße 22
40878 Ratingen (DE)**

Remarks:
The complete document including Reference
Table(s) and the Sequence Listing(s) can be
downloaded from the EPO website

(54) **MUTATED LIPASE**

(57) The present invention relates to a mutated lipase. The present invention further relates to a gene encoding the lipase, and a vector and a host cell comprising the gene. In addition, the present invention further relates to use of the lipase.

**EP 4 640 829 A1**

## Description

Technical Field

The present invention relates to the field of biotechnology, and more specifically to a mutant lipase. It also relates to a gene encoding the enzyme, and a vector and a host cell comprising the gene. Moreover, it relates to the use of the enzyme.

Background

As an important petroleum refining product, diesel occupies a high share in the composition of the fuel of various countries and has become an important power fuel. With the acceleration of the dieselization trend of vehicles worldwide, the demand for diesel will become larger and larger in the future. The increasing depletion of petroleum resources and the improvement of people's environmental awareness have greatly promoted the development of diesel which replaces fuels in various countries around the world. Especially since the 1990s, biodiesel has attracted attention from various countries for its superior environmental protection performance. At present, the annual production of new cars in the world is about 50 million, and the number of cars in the world is about 750 million (including motorcycles). With the rapid development of the automobile industry, the use of gasoline and diesel has increased with the increase in the number of cars, which has also brought about problems such as automobile exhaust pollution.

It is well-known that diesel molecules are composed of about 15 carbon chains. Studies have found that plant oil molecules are generally composed of 14 to 18 carbon chains, which is similar to the number of carbon atoms in diesel molecules. Therefore, biodiesel is a new type of fuel processed from renewable plant oil such as rapeseed. Compared with conventional diesel, biodiesel has the following incomparable properties: (1) It has excellent environmental protection characteristics. The use of biodiesel can reduce air toxicity by 90% and reduce the cancer rate by 94%; carbon monoxide emissions are reduced by about 10% compared with diesel. (2) It has good low-temperature engine starting performance. (3) It has good lubrication performance. (4) It has good safety performance. (5) It has good fuel performance. (6) It has renewable properties. The carbon dioxide emitted when biodiesel is burned is much less than the carbon dioxide absorbed during the growth of the plant, thereby improving the global warming caused by carbon dioxide emissions, a major environmental problem that is harmful to mankind. Therefore, biodiesel is a truly green diesel.

Currently, United States and some countries and regions in Europe and Asia have begun to establish commercial biodiesel production bases and widely use biodiesel as an alternative fuel. This is mainly because the United States has soybeans, Europe has rapeseed, and Asia has palm oil. The sustainable development of these agricultural industries requires finding ways to consume large quantities of vegetable oil other than food and chemical industries, guaranteeing the consumption of biodiesel in legal form, and promoting the development of the biodiesel industry with subsidies.

At present, biodiesel is mainly produced by chemical methods, that is, animal and vegetable oil and low-carbon alcohols such as methanol or ethanol are subjected to transesterification reaction under acid or alkaline catalysts and high temperature (230-250°C) to generate corresponding fatty acid methyl esters or ethyl esters, which are then washed and dried to obtain biodiesel. Methanol or ethanol can be recycled in the production process, and the production equipment is the same as general oil production equipment. About 10% of glycerol can be produced as a by-product during the production process.

The main problem of biodiesel at present is its high cost. According to statistics, 75% of the cost of biodiesel production is the cost of raw materials. Therefore, the use of cheap raw materials and improving conversion to reduce costs are the key to whether biodiesel can be practical. The United States has begun to study plants with high oil content through genetic engineering methods. Japan uses industrial waste oil and waste frying oil. Europe grows oil-rich crops on land that is not suitable for growing food. Asia uses waste oil and oil processing by-products for production.

However, chemical synthesis of biodiesel has the following disadvantages: the process is complex, alcohol must be in excess, and the subsequent process must have a corresponding alcohol recovery device, which consumes a lot of energy; the color is dark because the unsaturated fatty acids in the fat are easily deteriorated at high temperatures; the esterification products are difficult to recover and the cost is high; and waste alkali liquid is discharged during the production process.

To solve the above problems, people began to study the synthesis of biodiesel by bioenzyme, that is, using animal fat and low-carbon alcohols to undergo transesterification reactions with lipases to prepare the corresponding fatty acid methyl esters and ethyl esters. Enzymatic synthesis of biodiesel has the advantages of mild conditions, small alcohol dosage, and no pollution emissions. However, the main problems are: poor high temperature stability of enzymes, certain toxicity of short-chain alcohols to enzymes, and short life of enzymes. The by-products glycerol and water are difficult to recover, which not only inhibits product formation, but also glycerol is toxic to immobilized enzymes, shortening the life of immobilized enzymes.

The sequence of *Thermomyces dupontii* lipase (TDL) was published in 2012. The protein is 291 amino acids long, with a 22-amino acid signal peptide and a mature peptide of 269 amino acids. It has a typical lipase triplet catalytic domain structure of Ser168-Asp223-His280. TDL has a high preference for hydrolyzing C8 triglycerides at 50°C and pH 9.0. The conversion rate of biodiesel produced from waste cooking oil (WCO) is 91.6%.

In the applicant's patent application CN 2016112256785, the inventor obtained a TDL mutant EP14. At a protein concentration of 20 mg/ml, an addition amount of 0.5%, and a reaction time of 48 hours, its biodiesel conversion rate can reach 93%. However, it does not meet the biodiesel standard, that is, the conversion rate of 96.5%.

Therefore, there is still a need in the art for new lipases with high conversion rates for biodiesel conversion.

Summary of the invention

In the present invention, the inventors used the amino acid sequence of EP14 in CN2016112256785 as the starting sequence, introduced seven mutation sites D27R, G38A, D96E, D111A, G163K, D254S and A256T by site-directed mutagenesis, and obtained the mutant TDLm2. TDLm2 was incubated at 35°C and 80% methanol for 6 hours, and the residual enzyme activity was increased from 7.7% of EP14 to 72%.

A biodiesel reaction was carried out using fatty matter as a substrate, with a protein concentration of 20 mg/ml, an addition amount of 0.45%, and a reaction time of 24 hours. The conversion rate could reach 94%.

Based on TDLm2, three mutation sites L86I, I93L and M95F were introduced by site-directed mutagenesis to obtain mutant LM. Based on TDLm2, four mutation sites L86I, I93L, M95F and L211F were introduced to obtain mutant LMB.

A biodiesel reaction was carried out using fatty matter as a substrate, with a protein concentration of 20 mg/ml and an addition amount of 0.45%. The conversion rate could reach 94% after 8 hours of LM reaction. After 24 hours of reaction, the conversion rate could reach 96.5%. After 8 hours of reaction, the conversion rate of LMB reaction could reach 95%. After 24 hours of reaction, the conversion rate could reach 96.5%.

Based on LMB, the point mutation F95W was introduced to obtain the mutant LMBW; the point mutation F95Y was introduced to obtain the mutant LMBY.

A biodiesel reaction was carried out using fatty matter as a substrate, with a protein concentration of 20 mg/ml and an addition amount of 0.45%. The conversion rate could reach 92% after 8 hours of LMBW reaction. After 24 hours of reaction, the conversion rate could reach 96.6%. After 8 hours of LMBY reaction, the conversion rate could reach 96%. After 24 hours of reaction, the conversion rate could reach 96.6%.

The biodiesel conversion rates of the mutants TDLm2, LM, LMB, LMBW and LMBY obtained by the invention are greatly improved, and the amount of enzyme used and the reaction time can be reduced, thereby reducing the production cost of biodiesel.

Specifically, the present invention relates to the following aspects:

In one aspect, the present invention relates to a lipase comprising an amino acid sequence having a mutation relative to SEQ ID NO: 1 at one or more amino acid positions corresponding to positions 27, 38, 96, 111, 163, 254 and 256 of SEQ ID NO: 1. In one embodiment, the mutation is one or more of D27R, G38A, D96E, D111A, G163K, D254S and A256T.

In one embodiment, the lipase of the present invention further comprises an additional mutation at one or more amino acid positions corresponding to positions 86, 93, 95 and 211 of SEQ ID NO: 1. In one embodiment, the additional mutation is a mutation at position 95 of SEQ ID NO: 1, and one or more of L86I, I93L and L211F. In one embodiment, the additional mutation is one or more of L86I, I93L and M95F; L86I, I93L, M95F and L211F; L86I, I93L, M95W and L211F; or L86I, I93L, M95Y and L211F.

In one embodiment, the lipase of the present invention comprises an amino acid sequence selected from the group consisting of SEQ ID NO: 2, 4, 6, 8 and 10, or a sequence having at least 90%, or at least 95% or at least 99% sequence identity thereto.

As used herein, "percent identity" refers to the degree to which two optimally aligned DNA or protein segments remain unchanged throughout a comparison window of components such as nucleotide sequences or amino acid sequences. The "identity fraction" for the comparison segments of a test sequence and a reference sequence is the number of identical components shared by the two comparison segment sequences through the comparison window divided by the total number of sequence components in the reference segment, the total number being the smaller of the complete test sequence or the complete reference sequence. "Percent identity" ("identity %") is the identity fraction multiplied by 100.

In another aspect, the present invention relates to a method for preparing a lipase mutant, comprising introducing one or more mutations at amino acid positions of a starting lipase corresponding to positions 27, 38, 96, 111, 163, 254 and 256 of SEQ ID NO: 1. In one embodiment, the mutation is one or more of D27R, G38A, D96E, D111A, G163K, D254S and A256T. In one embodiment, the method further comprises introducing one or more mutations at amino acid positions of a starting lipase corresponding to positions 86, 93, 95 and 211 of SEQ ID NO: 1. In one embodiment, the mutation is one or more of L86I, I93L and M95F; L86I, I93L, M95F and L211F; L86I, I93L, M95W and L211F; or L86I, I93L, M95Y and L211F. In one embodiment, the sequence of the starting lipase is as shown in SEQ ID NO: 1.

In another aspect, the present invention relates to a nucleic acid molecule selected from the group consisting of: (a) a nucleotide sequence encoding the above-mentioned lipase, or a sequence having at least 90%, at least 95% or at least 99% sequence identity thereto; and (b) a nucleotide sequence complementary to the nucleotide sequence described in (a), which may be partially or completely complementary. In one embodiment, the nucleic acid molecule comprises a nucleotide sequence selected from SEQ ID NO: 3, 5, 7, 9 and 11, or a sequence having at least 90%, at least 95% or at least 99% sequence identity thereto.

The skilled in the art will understand, due to the degeneracy of the genetic code, a variety of different nucleotide sequences can encode the same enzyme. In addition, it will be appreciated that the skilled in the art can use routine techniques to replace nucleotides that do not affect the enzymatic activity encoded by the nucleotide sequence of the present invention, which can reflect the codon bias of any specific host organism used to express the enzyme of the present invention.

The present invention also provides a vector comprising the nucleic acid molecule, and a host cell comprising the nucleic acid molecule or the vector.

"Vector" refers to an extrachromosomal element that usually carries a gene that is not a part of the central metabolism of a cell, and is often in the form of a circular double-stranded DNA molecule. Such elements may be autonomously replicating sequences, genomic integration sequences, phage or nucleotide sequences, linear or circular single- or double-stranded DNAs or RNAs from any source, in which many of the nucleotide sequences have been spliced or recombined into specific constructs that are capable of introducing the promoter fragments and DNA sequences of the selected gene products together with appropriate 3' untranslated sequences into a cell.

Genes and gene products encoding the lipases of the invention can be expressed in heterologous host cells, such as bacterial cells, fungal cells, such as yeast cells, mammalian cells, insect cells and plant cells. Heterologous host cells for expressing the nucleic acid molecules of the invention can be microbial hosts in fungal or bacterial families and grow over a wide range of temperature, pH, and solvent tolerance. For example, it is contemplated that any bacteria, yeast, and filamentous fungi may be suitable hosts for expression of the nucleic acid molecules of the invention. Examples of host strains include, but are not limited to, bacterial, fungal or yeast species such as *Pichia, Aspergillus, Trichoderma, Saccharomyces, Phaffia , Kluyveromyces, Yarrowia, Candida, Hansenula, Salmonella, Bacillus, Acinetobacter, Zymomonas, Agrobacterium, Erythrobacter, Chlorobium, Chromatium, Flavobacterium, Cytophaga, Rhodobacter, Rhodococcus, Streptomyces, Brevibacterium, Corynebacteria, Mycobacterium, Deinococcus, Escherichia, Erwinia, Pantoea, Pseudomonas, Sphingomonas, Methylomonas, Methylobacter, Methylococcus, Methylosinus, Methylomicrobium, Methylocystis, Alcaligenes, Synechocystis, Synechococcus, Anabaena, Thiobacillus, Methanobacterium, Klebsiella and Myxococcus species.* In one embodiment, the host cell is a fungal cell. In one embodiment, the host cell is *Pichia pastoris.*

Vectors useful for transforming the host cells described above are well known in the art. Typically, vectors contain sequences that direct transcription and translation of the related genes, selectable markers, and sequences that allow autonomous replication or chromosomal integration. A suitable vector contains a 5' region of the gene that controls transcription initiation and a 3' region of the DNA fragment that controls transcription termination.

In one aspect, the present invention also relates to a method for producing a lipase, comprising expressing a nucleic acid molecule encoding the lipase of the present invention in a host cell, and recovering the resulting polypeptide.

A variety of culture methods can be used to prepare the enzymes of the invention. For example, large-scale production of specific gene products from recombinant microbial hosts can be performed by batch, fed-batch, and continuous culture methods.

Batch and fed-batch culture methods are commonly used and well known in the art, and examples can be found in: Thomas D. Brock in Biotechnology: A Textbook of Industrial Microbiology, Second Edition, Sinauer Associates, Inc., Sunderland, MA (1989)), and Deshpande, Mukund V., (Appl. Biochem. Biotechnol., 36: 227-234 (1992).

Commercial production of the enzymes of the invention can also be carried out by continuous culture. Continuous culture is an open system in which conditioned media are continuously added to the bioreactor and equal amounts of conditioned media are simultaneously removed for processing. Continuous culture generally maintains cells at a constant high liquid density where the cells are primarily in the logarithmic phase of growth. Alternatively, continuous culture can be performed with immobilized cells, wherein carbon and nutrients are continuously added and valuable products, by-products or waste products are continuously removed from the cell pellet.

Cell immobilization can be performed using a wide range of solid supports consisting of natural and/or synthetic materials.

Recovery of the desired enzyme from batch fermentation, fed-batch fermentation, or continuous culture can be accomplished by any method known to those skilled in the art. For example, when an enzyme is produced intracellularly, the cell slurry is separated from the culture medium by centrifugation or membrane filtration, optionally washed with water or an aqueous buffer of the desired pH. Then the cell slurry in the aqueous buffer of the desired pH is suspended to be homogenized to produce a cell extract containing the required enzyme.

The present invention also relates to a composition comprising the lipase of the invention, or a fermentation broth, fermentation supernatant and/or fermentation concentrate of the host cell of the invention. The enzyme composition of the present invention may be in any form suitable for use, for example, crude fermentation broth with or without cell removal, cell lysate with or without cell debris, semi-purified or purified enzyme composition, or host cells as a source of enzymes. The enzyme composition may be a dry powder or granule, a dust-free granule, a liquid, a stabilized liquid or a stabilized protected enzyme. The liquid enzyme composition can be stabilized according to established processes, for example by adding stabilizers such as sugars, sugar alcohols or other polyols, and/or lactic acid or other organic acids. The enzyme composition of the present invention may also include other lipases, for example: Lipex Evity 200L, Lipozyme 435, Lipase A "Amano" 6, Lipase AY "Amano" 30SD, Lipase G "Amano" 50, Lipase R "Amano", Lipase DF "Amano" 15, Lipase MER "Amano", Newlase F.

The present invention further relates to the fermentation broth, fermentation supernatant or fermentation concentrate of the host cell of the present invention.

The present invention also relates to use of the lipase of the present invention in converting fatty matter into biodiesel. When the lipase of the present invention is used for biodiesel conversion, compared with the lipase of the prior art, the amount of enzyme used and the reaction time can be reduced, thereby reducing the production cost of biodiesel.

The present invention also relates to a method for preparing biodiesel, comprising incubating raw material oil, a low-carbon alcohol, and the lipase or the composition or the fermentation broth, the fermentation supernatant, and/or the fermentation concentrate of the host cell of the present invention together to produce corresponding fatty acid ester. In one embodiment, the raw material oil is selected from oil of oil crops, wild oil plants and aquatic plants such as engineered microalgae, animal fat and waste cooking oil, and the low-carbon alcohol is selected from methanol and ethanol.

BRIEF DESCRIPTION OF THE DRAWINGS

FIG.1 shows the results of the comparison of methanol tolerance between TDLm2 of the present invention and EP14 of the prior art.

FIG.2 shows the results of measuring the concentrations of PTDLm2, PLM, PLMB, PLMBW and PLMBY in the fermentation broth using a modified Bradford protein concentration determination kit.

DETAILED DESCRIPTION

Experimental Materials

1. Experimental strains and plasmids

Strain: *Pichia pastoris* M316 (prepared by the applicant's previous work, deposit number CGMCC19221, see patent application number WO2020135763A1).

Plasmid: pET-pAOm-plc plasmid, synthesized by Shanghai Sangon Biotech Co., Ltd. The synthesis steps were as follows: the pAO815 vector sequence was prepared, which carried the encoding sequence of the PLC mutant sequence in the patent CN201680072289.5 "Highly efficient zinc-independent phospholipase C mutant" (SEQ ID NO: 7 of CN201680072289.5, Xaa is His) in the expression cassette, and was ligated into the pET-28C vector.

The encoding sequence of the PLC mutant sequence in the above patent CN201680072289.5 "Highly efficient zinc-independent phospholipase C mutant" is:

tggtcagctgaggacaagcataaggaaggtgtgaatagtcacttatggatcgtgaaccgtgccattgatataatgtctaggaatacaactctggt
taagcaagatagagttgctcaattgaatgaatggcgtacagagctagagaatggcatctacgctgctgatcatgaaaacccctattacgatgac
agtaccttcgcttctcacttttacgatccagacaacggaaagacatatatcccattcgccaagcaagctaaggagactggagctaagtacttcaa
gttggctggagagtcatacaagaataaagacatgaagcaggccttctttttatcttgggttgtcattgcattatttgggcgatgtcaaccaacctatg
catgccgcatccttacggacctgtcctatccacaggggttttcactccaagtacgagaactttgtcgatactattaaagacaactacaaagttacc
gatgggaacggatattggaattggaaaggcaccaaccctgaagaatggattcacggtgcagcagtagttgcaaaacaggactactctggaat
tgtcaatgacaataccaaagattggtttgtgaaagccgcagtctcccaggaatatgcagataaatggagagctgaagttacacctatgactggt
aaacgactaatggatgcccaaagagttactgctggtacattcaattatggttcgacacttacggtgacaggtaa (SEQ ID NO:12).

2. Culture media and solutions

YPD liquid medium: 1% yeast extract, 2% peptone, 2% glucose.

YPD solid medium: 2% agar was added into LB liquid medium.

BMMY-olive oil screening medium: Component A: 1% yeast extract, 2% peptone, 1.34% yeast nitrogen base (YNB) containing ammonium sulfate but not amino acids, 1% glycerol, $4 \times 10^{-5}$%D-biotin (added after sterilization), 0.1M citric acid-sodium citrate buffer pH 6.6, 2% agar. Component B: Olive oil substrate solution: 150ml of 4% PVA solution was taken, added with 50ml of olive oil, emulsified with a high-speed homogenizer at 8000rpm for 3min, paused for 1min and then emulsified for 3min to prepare the substrate solution. 100ml of sterilized component A was mixed with 12ml of component B, added with 1ml of 0.1% rhodamine B and 2% methanol.

BMGY liquid medium: 1% yeast extract, 2% peptone, 1.34% yeast nitrogen base (YNB) containing ammonium sulfate but not amino acids, 1% glycerol, $4 \times 10^{-5}$% D-biotin, 0.1M citric acid-sodium citrate buffer pH 6.6.

BMMY liquid medium: 1% yeast extract, 2% peptone, 1.34% yeast nitrogen base (YNB) containing ammonium sulfate but not amino acids, 2% methanol, $4 \times 10^{-5}$% D-biotin, 0.1M citric acid-sodium citrate buffer pH 6.6.

The modified Bradford protein concentration determination kit was purchased from Shanghai Sangon Biotech Co., Ltd.

PCR enzyme: PrimeSTAR®HS DNA Polymerase (purchased from TakaraBio (Dalian) Co., Ltd.).

Example 1: Expression of mutant TDLm2 in *Pichia pastoris* and preparation of enzyme solution

The amino acid sequence of the mature peptide of EP14 in CN 2016112256785 is as follows:

DVSQDLFDQFNLFAQYSAAAYCAKNNDAPAGAIVTCRGSICPEVEKADATFLYSFEDSGV
GDVTGFLALDNTNRLIVLSFRGSRSLENWIGNINMDLKGIDDICSGCKGHDGFTSSWRSVA
NTLTQQVQNAVREHPDYRVVFTGHSLGGALATVAGASLRGNGYDIDVFSYGAPRVGNRA
FAEFLTAQTGGTLYRITHTNDIVPRLPPReLGYSHSSPEYWITSGTLVPVRRRDIVKVEGIDS
TDGNNQPNTPDIAAHFWYFGSIGTCL(EP14, SEQ ID NO:1).

Seven mutation sites, D27R, G38A, D96E, D111A, G163K, D254S and A256T, were introduced into SEQ ID NO: 1 to obtain the amino acid sequence

DVSQDLFDQFNLFAQYSAAAYCAKNNRAPAGAIVTCRASICPEVEKADATFLYSFEDSGV
GDVTGFLALDNTNRLIVLSFRGSRSLENWIGNINMELKGIDDICSGCKGHAGFTSSWRSVA
NTLTQQVQNAVREHPDYRVVFTGHSLGGALATVAGASLRGNKYDIDVFSYGAPRVGNRA
FAEFLTAQTGGTLYRITHTNDIVPRLPPRELGYSHSSPEYWITSGTLVPVRRRDIVKVEGIDS
TDGNNQPNTPSITAHFWYFGSIGTCL (TDLm2, SEQ ID NO: 2).

The DNA sequence was designed based on the codon preference of *Pichia pastoris* and the prepropeptide sequence of a-factor (derived from the DNA sequence of the commercial vector pPIC9K) was added. The following DNA encoding sequence was obtained:

ATGAGATTTCCTTCAATTTTTACTGCAGTTTTATTCGCAGCATCCTCCGCATTAGCTGCT
CCAGTCAACACTACAACAGAAGATGAAACGGCACAAATTCCGGCTGAAGCTGTCATC
GGTTACTCAGATTTAGAAGGGGATTTCGATGTTGCTGTTTTGCCATTTTCCAACAGCAC
AAATAACGGGTTATTGTTTATAAATACTACTATTGCCAGCATTGCTGCTAAAGAAGAAG
GGGTATCTCTTGAGAAAGAGAGGCTGAAGCTGATGTTTCTCAAGATTTGTTTGATCA
ATTTAATTTGTTTGCTCAATATTCTGCTGCAGCTTATTGTGCTAAAAATAATAGGGCTCCT
GCTGGTGCTATTGTTACTTGTAGAGCTTCTATTTGTCCTGAAGTTGAAAAAGCTGATGC
TACTTTTTTGTATTCTTTTGAAGATTCTGGTGTTGGTGATGTTACTGGTTTTTTGGCTTTG
GATAATACTAATAGATTGATTGTTTTGTCTTTTAGAGGTTCTAGGTCATTAGAAAATTGG
ATTGGTAATATTAACATGGAATTAAAAGGTATTGATGATATTTGTTCTGGTTGTAAAGGT
CATGCTGGTTTTACTTCTTCTTGGAGGTCTGTTGCTAATACTTTGACTCAACAAGTTCAA
AATGCTGTTAGAGAACATCCTGATTATAGAGTTGTTTTTACTGGTCATTCTTTGGGTGGT
GCTTTGGCTACTGTTGCTGGTGCTTCTTTGAGAGGTAATAAATATGATATTGATGTTTTTT
CTTATGGTGCTCCAAGAGTTGGTAATAGAGCTTTTGCTGAATTTTTGACTGCTCAAACT
GGTGGTACTTTGTATAGAATTACTCATACTAATGATATTGTTCCAAGATTGCCACCAAGA
GAATTGGGTTATTCTCATTCTTCTCCTGAATATTGGATTACTTCTGGTACTTTGGTTCCTG
TTAGAAGGAGAGATATTGTTAAAGTTGAAGGTATTGATTCTACTGATGGTAATAATCAAC
CAAATACTCCATCTATTACTGCTCATTTTTGGTATTTTGGTTCTATTGGTACTTGTTTGtaa
(encoding sequence of TDLm2, SEQ ID NO: 3).

The DNA sequence was sent to Suzhou Genewiz Biotechnology Co., Ltd. for full gene synthesis and was cloned into pET-pAOm-plc vector through the SacII and EcoRI sites to obtain the plasmid p-TDLm2.

p-TDLm2 was linearized with SalI, and competent cells of *Pichia pastoris* GS 115 were prepared by LiAC method. Then, the linearized p-TDLm2 fragment was transformed into GS115 competent cells by electroporation. The transformants were coated on a MGYS plate and cultured at 30°C for 3 days. A large number of single clones on the plate were picked on a BMMY-olive oil screening plate, and positive clones with the best activity performance were picked out and named PTDLm2.

PTDLm2 and EP14 strains (EP14 strain was obtained with the encoding sequence of EP14 enzyme (SEQ ID NO: 6 in patent application CN 201611225678.5) by the same construction method as PTDLm2 strain) were first activated in liquid YPD, inoculated in BMGY, cultured overnight at 30°C, 220rpm, and then transferred to BMGY medium (the initial OD600 was 6), and initially induced with 2% methanol. 1% methanol was added after 24h and 32h respectively, and 1% methanol was added after 48h and 56h respectively. After 72h, the sample was taken, and concentrated with an ultrafiltration tube with a molecular weight cutoff of 10KD to make the protein concentration reach 30mg/ml.

The enzyme solutions of pTDLm2 and EP14 were adjusted to 30 mg/ml. The residual enzyme activity was measured after incubated at 35°C for 6 hours at a methanol concentration of 80%. The experiments were set up in 2 parallels and repeated twice. The enzyme activity of the untreated enzyme solution was used as a control, which had the relative enzyme activity recorded as 100%. The enzyme activity determination method was as follows:

150 ml of 4% PVA solution was taken, added with 50 ml of olive oil, and emulsified with a high-speed homogenizer at 8000 rpm for 3 minutes. After a 1-minute pause, it was emulsified for another 3 minutes to prepare the substrate solution (this solution must be prepared and used immediately).

sample bottle (100ml conical bottle)     blank bottle (100ml conical bottle)

↓     ↓

each added with substrate solution 4.00ml + pH8.0 Tris-HCl buffer 2.00ml + distilled water 3.00ml

↓     ↓

the sample bottle and the blank bottle were both placed in a 40°C constant temperature water bath for 5 minutes

↓

1.00 ml of enzyme solution (fermentation supernatant) was added into each sample bottle

↓

immediately mixed and timing started, incubated at 40°C for 15 min of reaction

↓     ↓

1. the sample bottle was added with 10ml of 95% ethanol, shaken well, and the reaction was stopped     2. the blank bottle was added with 10ml of 95% ethanol, then added with 1ml of enzyme solution and shaken well

↓

3-5 drops of phenolphthalein were added to each of the blank and sample bottles as an indicator.

↓

0.05 mol/L NaOH standard solution was used to titrate the free fatty acids produced by hydrolysis

↓

pH indicated the end point, and the volume of NaOH consumed by the titration of the blank and the sample was recorded respectively

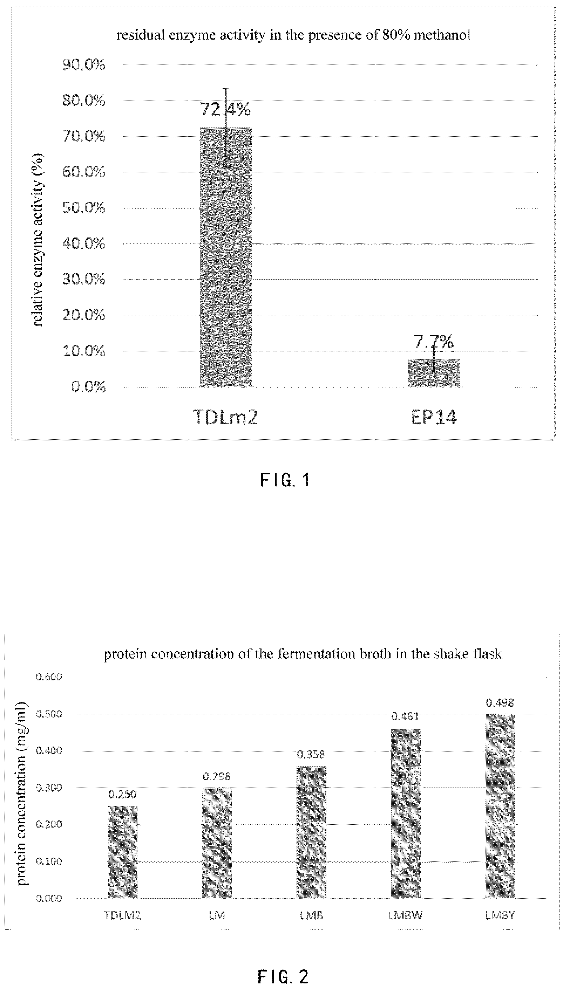

The residual enzyme activities of pTDLm2 and EP14 after incubation at 35°C for 6 hours in the presence of 80% methanol are shown in Figure 1.

Example 2: test of TDLm2 biodiesel reaction

Biodiesel preparation method:

1. Raw materials: Fatty matter (50%-60% palmitic acid, 40%-50% palmitic acid methyl ester, specifically, palmitic acid (purchased from Shanghai Aladdin Biochemical Technology Co., Ltd.) and palmitic acid methyl ester (purchased from Shanghai Aladdin Biochemical Technology Co., Ltd.) were mixed at a ratio of 5-6:4-5. In the following examples, the specific Fatty matter used was obtained by mixing in a ratio of 6:4. Methanol was purchased from Sinopharm Group.
Reaction system and reaction conditions:
Initial reaction system: Fatty matter 1ml + methanol 150ul + enzyme solution 4.5ul (protein concentration was 20mg/ml) + water 30ul
After methanol and Fatty matter were evenly mixed, enzyme solution and water were added. The sample was placed in a metal bath for incubation at 35°C and 1000rpm. After 2.5 hours of reaction, 100ul of methanol was added. After 4.5 hours of reaction, 50ul of methanol was added. After 8 hours and 24 hours of reaction, the acid values were measured respectively, and the free fatty acid (FFA)% was calculated.
2.FFA test method: The oil sample must be liquid and evenly mixed before measurement.
3. Approximately 0.2 g of oil sample were weighed, accurated to 0.001 g, and placed in a 50 mL centrifuge tube.
4. 10 mL of neutral isopropanol was added (several drops of 1% phenolphthalein indicator were added). The oil sample was fully dissolved by shaking (heated if necessary).

5. 0.05 mol/L potassium hydroxide standard solution was used to titrate while shaken until pink appeared. If the color did not fade within 30 seconds, it was the titration endpoint.

6. The volume (V) of potassium hydroxide consumed in the titration was recorded and the result was calculated.

Representation of analysis results

$$\text{Acid value} = V \times c \times 56.1/m$$

Wherein:

V - volume of potassium hydroxide solution used, ml;
c - the exact concentration of potassium hydroxide standard solution used, mol/L;
M - sample mass, g;
56.1-molar mass of potassium hydroxide, g/mol.
FFA% = acid value/2 $\times$ 100%

The results of biodiesel production of TDLm2 and EP14 are shown in the following table:

| Reaction time | FFA%/ conversion rate(TDLm2) | FFA%/conversion rate (EP14) |
|---|---|---|
| 8h | 15%/85% | 44%/56% |
| 24h | 6%/94% | 42%/58% |

It can be seen that TDLm2 of the present invention leads to a significantly improved biodiesel conversion rate compared to EP14.

Example 3: Expression of mutants LM and LMB in *Pichia pastoris* and preparation of enzyme solution

The amino acid sequence of the mature peptide of TDLm2 was introduced with three mutation sites, L86I, I93L and M95F to obtain the following amino acid sequence:

DVSQDLFDQFNLFAQYSAAAYCAKNNRAPAGAIVTCRASICPEVEKADATFLYSFEDSGV
GDVTGFLALDNTNRLIVLSFRGSRSIENWIGNLNFELKGIDDICSGCKGHAGFTSSWRSVA
NTLTQQVQNAVREHPDYRVVFTGHSLGGALATVAGASLRGNKYDIDVFSYGAPRVGNRA

FAEFLTAQTGGTLYRITHTNDIVPRLPPRELGYSHSSPEYWITSGTLVPVRRRDIVKVEGIDS
TDGNNQPNTPSITAHFWYFGSIGTCL (LM, SEQ ID NO: 4).

The DNA sequence was designed based on the codon preference of *Pichia pastoris* and the prepropeptide sequence of a-factor (derived from the DNA sequence of the commercial vector pPIC9K) was added. The following DNA sequence was obtained:

ATGAGATTTCCTTCAATTTTTACTGCAGTTTTATTCGCAGCATCCTCCGCATTAGCTGCT
CCAGTCAACACTACAACAGAAGATGAAACGGCACAAATTCCGGCTGAAGCTGTCATC
GGTTACTCAGATTTAGAAGGGGATTTCGATGTTGCTGTTTTGCCATTTTCCAACAGCAC
AAATAACGGGTTATTGTTTATAAATACTACTATTGCCAGCATTGCTGCTAAAGAAGAAG
GGGTATCTCTTGAGAAAGAGAGGCTGAAGCTGATGTTTCTCAAGATTTGTTTGATCA
ATTTAATTTGTTTGCTCAATATTCTGCTGCAGCTTATTGTGCTAAAAATAATAGGGCTCCT
GCTGGTGCTATTGTTACTTGTAGAGCTTCTATTTGTCCTGAAGTTGAAAAAGCTGATGC
TACTTTTTTGTATTCTTTTGAAGATTCTGGTGTTGGTGATGTTACTGGTTTCTTAGCATTG
GATAATACTAATAGATTGATTGTTTTGTCATTCAGAGGATCTAGGTCTATTGAAAATTGG
ATCGGTAATTTGAACTTTGAATTGAAAGGTATTGATGATATTTGTTCTGGTTGTAAAGGT
CATGCTGGTTTTACTTCTTCTTGGAGGTCTGTTGCTAATACTTTGACTCAACAAGTTCAA
AATGCTGTTAGAGAACATCCTGATTATAGAGTTGTTTTTACTGGTCATTCTTTGGGTGGT
GCTTTGGCTACTGTTGCTGGTGCTTCTTTGAGAGGTAATAAATATGATATTGATGTTTTTT
CTTATGGTGCTCCAAGAGTTGGTAATAGAGCTTTTGCTGAATTTTTGACTGCTCAAACT
GGTGGTACTTTGTATAGAATTACTCATACTAATGATATTGTTCCAAGATTGCCACCAAGA
GAATTGGGTTATTCTCATTCTTCTCCTGAATATTGGATTACTTCTGGTACTTTGGTTCCTG
TTAGAAGGAGAGATATTGTTAAAGTTGAAGGTATTGATTCTACTGATGGTAATAATCAAC
CAAATACTCCATCTATTACTGCTCATTTTTGGTATTTTGGTTCTATTGGTACTTGTTTGtaa
(encoding sequence of LM, SEQ ID NO: 5).

The DNA sequence was sent to Suzhou Genewiz Biotechnology Co., Ltd. for full gene synthesis and was cloned into pET-pAOm-plc vector through the SacII and EcoRI sites to obtain the plasmid p-LM.
The amino acid sequence of the mature peptide of TDLm2 was introduced with four mutation sites, L86I, I93L, M95F and L211F to obtain the following amino acid sequence:

DVSQDLFDQFNLFAQYSAAAYCAKNNRAPAGAIVTCRASICPEVEKADATFLYSFEDSGV
GDVTGFLALDNTNRLIVLSFRGSRSIENWIGNLNFELKGIDDICSGCKGHAGFTSSWRSVA
NTLTQQVQNAVREHPDYRVVFTGHSLGGALATVAGASLRGNKYDIDVFSYGAPRVGNRA
FAEFLTAQTGGTLYRITHTNDIVPRLPPREFGYSHSSPEYWITSGTLVPVRRRDIVKVEGIDS
TDGNNQPNTPSITAHFWYFGSIGTCL(LMB, SEQ ID NO:6)

The DNA sequence was designed based on the codon preference of *Pichia pastoris* and the prepropeptide sequence of a-factor (derived from the DNA sequence of the commercial vector pPIC9K) was added. The following DNA sequence was obtained:

ATGAGATTTCCTTCAATTTTTACTGCAGTTTTATTCGCAGCATCCTCCGCATTAGCTGCT
CCAGTCAACACTACAACAGAAGATGAAACGGCACAAATTCCGGCTGAAGCTGTCATC

GGTTACTCAGATTTAGAAGGGGATTTCGATGTTGCTGTTTTGCCATTTTCCAACAGCAC
AAATAACGGGTTATTGTTTATAAATACTACTATTGCCAGCATTGCTGCTAAAGAAGAAG
GGGTATCTCTTGAGAAAAGAGAGGCTGAAGCT<u>GAT</u>GTTTCTCAAGATTTGTTTGATCA
ATTTAATTTGTTTGCTCAATATTCTGCTGCAGCTTATTGTGCTAAAAATAATAGGGCTCCT
GCTGGTGCTATTGTTACTTGTAGAGCTTCTATTTGTCCTGAAGTTGAAAAAGCTGATGC
TACTTTTTTGTATTCTTTTGAAGATTCTGGTGTTGGTGATGTTACTGGTTTCTTAGCATTG
GATAATACTAATAGATTGATTGTTTTGTCATTCAGAGGATCTAGGTCTATTGAAAATTGG
ATCGGTAATTTGAACTTTGAATTGAAAGGTATTGATGATATTTGTTCTGGTTGTAAAGGT
CATGCTGGTTTTACTTCTTCTTGGAGGTCTGTTGCTAATACTTTGACTCAACAAGTTCAA
AATGCTGTTAGAGAACATCCTGATTATAGAGTTGTTTTTACTGGTCATTCTTTGGGTGGT
GCTTTGGCTACTGTTGCTGGTGCTTCTTTGAGAGGTAATAAATATGATATTGATGTTTTTT
CTTATGGTGCTCCAAGAGTTGGTAATAGAGCTTTTGCTGAATTTTTGACTGCTCAAACT
GGTGGTACTTTGTATAGAATTACTCATACTAATGATATTGTTCCAAGATTGCCACCAAGA
GAATTTGGTTATTCTCATTCTTCTCCTGAATATTGGATTACTTCTGGTACTTTGGTTCCTG
TTAGAAGGAGAGATATTGTTAAAGTTGAAGGTATTGATTCTACTGATGGTAATAATCAAC
CAAATACTCCATCTATTACTGCTCATTTTTGGTATTTTGGTTCTATTGGTACTTGTTTGtaa
(encoding sequence of LMB, SEQ ID NO: 7).

The DNA sequence was sent to Suzhou Genewiz Biotechnology Co., Ltd. for full gene synthesis and was cloned into pET-pAOm-plc vector through the SacII and EcoRI sites to obtain the plasmid p-LMB.

p-LM and p-LMB were linearized with SalI, and competent cells of *Pichia pastoris* GS115 were prepared by the LiAC method. The linearized p-LM and p-LMB fragments were then transformed into GS115 competent cells by electroporation. The transformants were coated on a MGYS plate and cultured at 30°C for 3 days. A large number of single clones on the plate were picked on a BMMY-olive oil screening plate, and positive clones with the best activity performance were picked out and named PLM and PLMB.

PLM and PLMB strains were first activated in liquid YPD, inoculated in BMGY, cultured overnight at 30°C, 220rpm, and then transferred to BMGY medium (the initial OD600 was 6), and initially induced with 2% methanol. 1% methanol was added after 24h and 32h respectively, and 1% methanol was added after 48h and 56h respectively. After 72h, the sample was concentrated with an ultrafiltration tube with a molecular weight cutoff of 10KD to make the protein concentration reach 30mg/ml.

Example 4: biodiesel reaction tests of LM and LMB

The biodiesel preparation method is as described in Example 2.

The results of biodiesel production of LM and LMB are shown in the following table.

| Reaction time | FFA%/ conversion rate(LM) | FFA%/conversion rate (LMB) |
|---|---|---|
| 8h | 5%/95% | 4%/96% |
| 24h | 3.4%/96.6% | 3.4%/96.6% |

It can be seen that the LM and LMB of the present invention lead to a further improved biodiesel conversion rate compared to TDLm2.

Example 5: Expression of mutants LMBW and LMBY in *Pichia pastoris* and preparation of enzyme solution

The amino acid sequence of the mature peptide of LMB was introduced with the mutation F95W at amino acid position 95 to obtain the following amino acid sequence:

DVSQDLFDQFNLFAQYSAAAYCAKNNRAPAGAIVTCRASICPEVEKADATFLYSFEDSGV
GDVTGFLALDNTNRLIVLSFRGSRSIENWIGNLNWELKGIDDICSGCKGHAGFTSSWRSVA
NTLTQQVQNAVREHPDYRVVFTGHSLGGALATVAGASLRGNKYDIDVFSYGAPRVGNRA
FAEFLTAQTGGTLYRITHTNDIVPRLPPREFGYSHSSPEYWITSGTLVPVRRRDIVKVEGIDS
TDGNNQPNTPSITAHFWYFGSIGTCL(LMBW, SEQ ID NO:8) .

The DNA sequence was designed based on the codon preference of *Pichia pastoris* and the prepropeptide sequence of a-factor (derived from the DNA sequence of the commercial vector pPIC9K) was added. The following DNA encoding sequence was obtained:

ATGAGATTTCCTTCAATTTTTACTGCAGTTTTATTCGCAGCATCCTCCGCATTAGCTGCT
CCAGTCAACACTACAACAGAAGATGAAACGGCACAAATTCCGGCTGAAGCTGTCATC
GGTTACTCAGATTTAGAAGGGGATTTCGATGTTGCTGTTTTGCCATTTTCCAACAGCAC
AAATAACGGGTTATTGTTTATAAATACTACTATTGCCAGCATTGCTGCTAAAGAAGAAG
GGGTATCTCTTGAGAAAAGAGAGGCTGAAGCTGATGTTTCTCAAGATTTGTTTGATCA
ATTTAATTTGTTTGCTCAATATTCTGCTGCAGCTTATTGTGCTAAAAATAATAGGGCTCCT
GCTGGTGCTATTGTTACTTGTAGAGCTTCTATTTGTCCTGAAGTTGAAAAAGCTGATGC
TACTTTTTTGTATTCTTTTGAAGATTCTGGTGTTGGTGATGTTACTGGTTTTTTGGCTTTG
GATAATACTAATAGATTGATTGTTTTGTCTTTTAGAGGATCTAGGTCAATTGAAAACTGG
ATTGGTAATTTGAATTGGGAATTGAAAGGTATTGATGATATTTGTTCTGGTTGTAAAGGT
CATGCTGGTTTTACTTCTTCTTGGAGGTCTGTTGCTAATACTTTGACTCAACAAGTTCAA
AATGCTGTTAGAGAACATCCTGATTATAGAGTTGTTTTTACTGGTCATTCTTTGGGTGGT
GCTTTGGCTACTGTTGCTGGTGCTTCTTTGAGAGGTAATAAATATGATATTGATGTTTTTT
CTTATGGTGCTCCAAGAGTTGGTAATAGAGCTTTTGCTGAATTTTTGACTGCTCAAACT
GGTGGTACTTTGTATAGAATTACTCATACTAATGATATTGTTCCAAGATTGCCACCAAGA
GAATTTGGTTATTCTCATTCTTCTCCTGAATATTGGATTACTTCTGGTACTTTGGTTCCTG
TTAGAAGGAGAGATATTGTTAAAGTTGAAGGTATTGATTCTACTGATGGTAATAATCAAC
CAAATACTCCATCTATTACTGCTCATTTTGGTATTTTGGTTCTATTGGTACTTGTTTGtaa
(encoding sequence of LMBW, SEQ ID NO: 9)

The DNA sequence was sent to Suzhou Genewiz Biotechnology Co., Ltd. for full gene synthesis and was cloned into pET-pAOm-plc vector through the SacII and EcoRI sites to obtain the plasmid p-LMBW.

The amino acid sequence of the mature peptide of LMB was introduced with the mutation F95Y at amino acid position 95 to obtain the following amino acid sequence:

DVSQDLFDQFNLFAQYSAAAYCAKNNRAPAGAIVTCRASICPEVEKADATFLYSFEDSGV
GDVTGFLALDNTNRLIVLSFRGSRSIENWIGNLNYELKGIDDICSGCKGHAGFTSSWRSVA
NTLTQQVQNAVREHPDYRVVFTGHSLGGALATVAGASLRGNKYDIDVFSYGAPRVGNRA

FAEFLTAQTGGTLYRITHTNDIVPRLPPREFGYSHSSPEYWITSGTLVPVRRRDIVKVEGIDS
TDGNNQPNTPSITAHFWYFGSIGTCL (LMBY, SEQ ID NO:10).

The DNA sequence was designed based on the codon preference of *Pichia pastoris* and the prepropeptide sequence of a-factor (derived from the DNA sequence of the commercial vector pPIC9K) was added. The following DNA sequence was obtained:

```
ATGAGATTTCCTTCAATTTTTACTGCAGTTTTATTCGCAGCATCCTCCGCATTAGCTGCT
CCAGTCAACACTACAACAGAAGATGAAACGGCACAAATTCCGGCTGAAGCTGTCATC
GGTTACTCAGATTTAGAAGGGGATTTCGATGTTGCTGTTTTGCCATTTTCCAACAGCAC
AAATAACGGGTTATTGTTTATAAATACTACTATTGCCAGCATTGCTGCTAAAGAAGAAG
GGGTATCTCTTGAGAAAGAGAGGCTGAAGCTGATGTTTCTCAAGATTTGTTTGATCA
ATTTAATTTGTTTGCTCAATATTCTGCTGCAGCTTATTGTGCTAAAAATAATAGGGCTCCT
GCTGGTGCTATTGTTACTTGTAGAGCTTCTATTTGTCCTGAAGTTGAAAAAGCTGATGC
TACTTTTTTGTATTCTTTTGAAGATTCTGGTGTTGGTGATGTTACTGGTTTTTTGGCTTTG
GATAATACTAATAGATTGATTGTTTTGTCTTTTAGAGGATCTAGGTCAATTGAAAACTGG
ATTGGTAATTTGAACTATGAATTGAAAGGTATTGATGATATTTGTTCTGGTTGTAAAGGT
CATGCTGGTTTTACTTCTTCTTGGAGGTCTGTTGCTAATACTTTGACTCAACAAGTTCAA
AATGCTGTTAGAGAACATCCTGATTATAGAGTTGTTTTTACTGGTCATTCTTTGGGTGGT
GCTTTGGCTACTGTTGCTGGTGCTTCTTTGAGAGGTAATAAATATGATATTGATGTTTTTT
CTTATGGTGCTCCAAGAGTTGGTAATAGAGCTTTTGCTGAATTTTTGACTGCTCAAACT
GGTGGTACTTTGTATAGAATTACTCATACTAATGATATTGTTCCAAGATTGCCACCAAGA
GAATTTGGTTATTCTCATTCTTCTCCTGAATATTGGATTACTTCTGGTACTTTGGTTCCTG
TTAGAAGGAGAGATATTGTTAAAGTTGAAGGTATTGATTCTACTGATGGTAATAATCAAC
CAAATACTCCATCTATTACTGCTCATTTTTGGTATTTTGGTTCTATTGGTACTTGTTTGtaa
```
(encoding sequence of LMBY, SEQ ID NO: 11).

The DNA sequence was sent to Suzhou Genewiz Biotechnology Co., Ltd. for full gene synthesis and was cloned into pET-pAOm-plc vector through the SacII and EcoRI sites to obtain the plasmid p-LMBY.

p-LMBW and p-LMBY were linearized with SalI, and competent cells of *Pichia pastoris* GS 115 were prepared by the LiAC method. The linearized p-LMBW and p-LMBY fragments were then transformed into GS115 competent cells by electroporation. The transformants were coated on a MGYS plate and cultured at 30°C for 3 days. A large number of single clones on the plate were picked on a BMMY-olive oil screening plate, and positive clones with the best activity performance were picked out and named PLMBW and PLMBY.

PTDLm2, PLM, PLMB, PLMBW and PLMBY strains were first activated in liquid YPD, inoculated in BMGY, cultured overnight at 30°C, 220rpm, and then transferred to BMGY medium (the initial OD600 was 6), and initially induced with 2% methanol. 1% methanol was added after 24h and 32h respectively, and 1% methanol was added after 48h and 56h respectively. After 72h, the sample was concentrated with an ultrafiltration tube with a molecular weight cutoff of 10KD to make the protein concentration reach 30mg/ml.

The shake flask fermentation broth of PTDLm2, PLM, PLMB, PLMBW and PLMBY was taken and the protein concentration of the fermentation broth was determined using a modified Bradford protein concentration determination kit. The protein concentration of the fermentation broth of each strain is shown in Figure 2.

The shake flask fermentation protein yields of LMBW and LMBY were 0.461 mg/ml and 0.498 mg/ml, respectively, compared with 0.25 mg/ml of TDLm2, they increased by 84% and 99% respectively, compared with 0.358 mg/ml of LMB, they increased by 29% and 39% respectively.

Example 6: LMBW and LMBY biodiesel reaction tests

The biodiesel preparation method is as described in Example 2.

The results of biodiesel production of LMBW and LMBY are shown in the following table.

| Reaction time | FFA%/ conversion rate(LMBW) | FFA%/conversion rate (LMBY) |
|---------------|------------------------------|------------------------------|
| 8h | 8%/92% | 4%/96% |
| 24h | 3.4%/96.6% | 3.4%/96.6% |

After 24 hours of reaction, the FFA% of LMBW and LMBY can both be reduced to 3.4%, which meets the standard of biodiesel. Therefore, LMBW and LMBY have the advantage of cost saving compared with TDLM2, LM and LMB.

**Claims**

1. A lipase, comprising an amino acid sequence having a mutation relative to SEQ ID NO: 1 at one or more amino acid positions corresponding to positions 27, 38, 96, 111, 163, 254 and 256 of SEQ ID NO: 1; preferably, the mutation is one or more of D27R, G38A, D96E, D111A, G163K, D254S and A256T.

2. The lipase of claim 1, further comprising an additional mutation at one or more amino acid positions corresponding to positions 86, 93, 95 and 211 of SEQ ID NO: 1; preferably, the additional mutation is one or more of L86I, I93L and M95F; L86I, I93L, M95F and L211F; L86I, I93L, M95W and L211F; or L86I, I93L, M95Y and L211F.

3. The lipase of claim 1 or 2, comprising an amino acid sequence selected from the group consisting of SEQ ID NO: 2, 4, 6, 8 and 10, or a sequence having at least 90%, or at least 95% or at least 99% sequence identity thereto.

4. A nucleic acid molecule comprising:

    (a) a nucleotide sequence encoding the lipase of claim 1, or a sequence having at least 90%, at least 95%, or at least 99% sequence identity thereto; and
    (b) a nucleotide sequence complementary to the nucleotide sequence of (a),

    preferably, the nucleic acid molecule comprises a nucleotide sequence selected from SEQ ID NO: 3, 5, 7, 9 and 11, or a sequence having at least 90%, or at least 95% or at least 99% sequence identity thereto.

5. A vector comprising the nucleic acid molecule of claim 4.

6. A host cell comprising the nucleic acid molecule of claim 4 or the vector of claim 5.

7. The host cell of claim 6, wherein the host cell is selected from the group consisting of a bacterial cell, a fungal cell, a mammalian cell, an insect cell and a plant cell, preferably, wherein the host cell is a fungal cell, more preferably a *Pichia pastoris* cell.

8. A method for producing a lipase, comprising expressing a nucleic acid molecule encoding the lipase of any one of claims 1 to 3 in a host cell, and recovering the resulting polypeptide.

9. A composition comprising the lipase according to any one of claims 1 to 3 or the fermentation broth, fermentation supernatant and/or fermentation concentrate of the host cell according to claim 6 or 7.

10. The fermentation broth, fermentation supernatant or fermentation concentrate of the host cell according to claim 6 or 7.

11. Use of the lipase according to any one of claims 1 to 3, or the composition according to claim 9, or the fermentation broth, fermentation supernatant and/or fermentation concentrate of the host cell according to claim 6 or 7 in converting fatty matter into biodiesel.

12. A method for preparing a lipase mutant, comprising introducing one or more mutations at amino acid positions of a starting lipase corresponding to positions 27, 38, 96, 111, 163, 254 and 256 of SEQ ID NO: 1, preferably, the mutation is one or more of D27R, G38A, D96E, D111A, G163K, D254S and A256T; optionally, the method further comprises introducing one or more mutations at amino acid positions of a starting lipase corresponding to positions 86, 93, 95 and 211 SEQ ID NO: 1, preferably, the mutation is one or more of L86I, I93L and M95F; L86I, I93L, M95F and L211F; L86I, I93L, M95Y and L211F; or L86I, I93L, M95W and L211F; preferably, the sequence of the starting lipase is as shown in SEQ ID NO: 1.

13. A method for preparing biodiesel, comprising incubating raw material oil, a low-carbon alcohol and a lipase according to any one of claims 1 to 3 or a composition according to claim 9 or a fermentation liquid, a fermentation supernatant and/or a fermentation concentrate of a host cell according to claim 6 or 7 together to produce corresponding fatty acid ester, preferably, the raw material oil is selected from oil of oil crops, wild oil plants and aquatic plants such as engineered microalgae, animal fat and waste cooking oil, and the low-carbon alcohol is selected from methanol and ethanol.

FIG. 1

FIG. 2

# INTERNATIONAL SEARCH REPORT

| International application No. |
|---|
| **PCT/CN2023/138703** |

**A. CLASSIFICATION OF SUBJECT MATTER**

C12N9/20(2006.01)i; C12N15/55(2006.01)i; C12N15/63(2006.01)i; C12P7/649(2022.01)i

According to International Patent Classification (IPC) or to both national classification and IPC

**B. FIELDS SEARCHED**

Minimum documentation searched (classification system followed by classification symbols)

IPC: C12N C12P

Documentation searched other than minimum documentation to the extent that such documents are included in the fields searched

Electronic data base consulted during the international search (name of data base and, where practicable, search terms used)

CNABS, DWPI, SIPOABS, CNTXT, EPTXT, USTXT, WOTXT, PUBMED, CNKI, NCBI, EBI, STN, 中国专利生物序列检索系统, Chinese Patent Biological Sequence Retrieval System, 百度学术, Baidu Scholar: 申请人, 发明人, 脂肪酶, lipase, 突变体, mutant, 嗜热踝节菌, Thermomyces dupontii, Talaromyces thermophilus, D27R, G38A, D96E, D111A, G163K, D254S, A256T, 序列1-11

**C. DOCUMENTS CONSIDERED TO BE RELEVANT**

| Category* | Citation of document, with indication, where appropriate, of the relevant passages | Relevant to claim No. |
|---|---|---|
| X | WO 2016050661 A1 (NOVOZYMES A/S) 07 April 2016 (2016-04-07) claims 1, 6, 9, and 11-15, and description, pages 26-27 and 29 | 1, 4-10, 12 |
| Y | WO 2016050661 A1 (NOVOZYMES A/S) 07 April 2016 (2016-04-07) claims 1, 6, 9, and 11-15, and description, pages 26-27 and 29 | 13 |
| X | WO 2017091674 A1 (THE PROCTER & GAMBLE COMPANY) 01 June 2017 (2017-06-01) description, pages 12-14 | 1, 12 |
| Y | WO 2017091674 A1 (THE PROCTER & GAMBLE COMPANY) 01 June 2017 (2017-06-01) description, pages 12-14 | 13 |
| X | WO 2018231798 A1 (THE PROCTER & GAMBLE COMPANY) 20 December 2018 (2018-12-20) description, pages 6-7 | 1, 12 |
| Y | WO 2018231798 A1 (THE PROCTER & GAMBLE COMPANY) 20 December 2018 (2018-12-20) description, pages 6-7 | 13 |

| ✓ Further documents are listed in the continuation of Box C. | ✓ See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **27 February 2024** | **08 March 2024** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

**EP 4 640 829 A1**

## INTERNATIONAL SEARCH REPORT

International application No.

**PCT/CN2023/138703**

### C. DOCUMENTS CONSIDERED TO BE RELEVANT

| Category* | Citation of document, with indication, where appropriate, of the relevant passages | Relevant to claim No. |
|---|---|---|
| Y | CN 108239626 A (WILMAR (SHANGHAI) BIOTECHNOLOGY RESEARCH & DEVELOPMENT CENTER CO., LTD.) 03 July 2018 (2018-07-03) claims 1-7, and description, embodiment 2 | 13 |
| A | CN 108359655 A (LIU DANNI) 03 August 2018 (2018-08-03) abstract, and claims 1-9 | 1-13 |
| A | CN 104293744 A (ZHEJIANG UNIVERSITY OF TECHNOLOGY) 21 January 2015 (2015-01-21) abstract, and claims 1-6 | 1-13 |
| A | CN 106676084 A (ZHEJIANG UNIVERSITY OF TECHNOLOGY) 17 May 2017 (2017-05-17) abstract, and claims 1-10 | 1-13 |
| A | WO 2016091870 A1 (NOVOZYMES A/S) 16 June 2016 (2016-06-16) abstract, and claims 6-17 | 1-13 |
| A | WO 2017101801 A1 (WILMAR (SHANGHAI) BIOTECHNOLOGY RESEARCH & DEVELOPMENT CENTER CO., LTD.) 22 June 2017 (2017-06-22) claims 1-10 | 1-13 |
| A | DING, Xu et al. "Engineering of Talaromyces thermophilus lipase by altering its crevice-like binding site for highly efficient biocatalytic synthesis of chiral intermediate of Pregablin" *Bioorganic Chemistry,* Vol. 77, 30 April 2018 (2018-04-30), pages 330-338 abstract | 1-13 |

Form PCT/ISA/210 (second sheet) (July 2022)

**INTERNATIONAL SEARCH REPORT**

International application No.

**PCT/CN2023/138703**

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

   a. ☑ forming part of the international application as filed.

   b. ☐ furnished subsequent to the international filing date for the purposes of international search (Rule 13*ter*.1(a)),

   ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
Information on patent family members

International application No.

**PCT/CN2023/138703**

| Patent document cited in search report | | | Publication date (day/month/year) | Patent family member(s) | | | Publication date (day/month/year) |
|---|---|---|---|---|---|---|---|
| WO | 2016050661 | A1 | 07 April 2016 | EP | 3201305 | A1 | 09 August 2017 |
| | | | | EP | 3201305 | B1 | 17 July 2019 |
| | | | | US | 2021071112 | A1 | 11 March 2021 |
| | | | | US | 2017306269 | A1 | 26 October 2017 |
| | | | | US | 10865366 | B2 | 15 December 2020 |
| WO | 2017091674 | A1 | 01 June 2017 | JP | 2019502779 | A | 31 January 2019 |
| | | | | CA | 3002668 | A1 | 01 June 2017 |
| | | | | ZA | 201802566 | B | 29 January 2020 |
| | | | | JP | 2020128542 | A | 27 August 2020 |
| | | | | EP | 3173467 | A1 | 31 May 2017 |
| | | | | MX | 2018006475 | A | 28 September 2018 |
| WO | 2018231798 | A1 | 20 December 2018 | EP | 3415592 | A1 | 19 December 2018 |
| CN | 108239626 | A | 03 July 2018 | None | | | |
| CN | 108359655 | A | 03 August 2018 | None | | | |
| CN | 104293744 | A | 21 January 2015 | None | | | |
| CN | 106676084 | A | 17 May 2017 | None | | | |
| WO | 2016091870 | A1 | 16 June 2016 | EP | 3230442 | A1 | 18 October 2017 |
| | | | | EP | 3230442 | B1 | 22 May 2019 |
| | | | | US | 2017321197 | A1 | 09 November 2017 |
| | | | | US | 11268076 | B2 | 08 March 2022 |
| | | | | CA | 2965232 | A1 | 16 June 2016 |
| | | | | AR | 102950 | A1 | 05 April 2017 |
| | | | | US | 2022145273 | A1 | 12 May 2022 |
| | | | | MX | 2017007105 | A | 24 August 2017 |
| WO | 2017101801 | A1 | 22 June 2017 | ES | 2882357 | T3 | 01 December 2021 |
| | | | | EP | 3392336 | A1 | 24 October 2018 |
| | | | | EP | 3392336 | A4 | 15 May 2019 |
| | | | | EP | 3392336 | B1 | 23 June 2021 |
| | | | | DK | 3392336 | T3 | 12 July 2021 |
| | | | | US | 2018362942 | A1 | 20 December 2018 |
| | | | | US | 10738288 | B2 | 11 August 2020 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 2016112256785
- WO 2020135763 A1
- CN 201680072289
- CN 201611225678

**Non-patent literature cited in the description**

- **THOMAS D. BROCK**. Biotechnology: A Textbook of Industrial Microbiology. Sinauer Associates, Inc., 1989
- **DESHPANDE, MUKUND V.** *Appl. Biochem. Biotechnol.*, 1992, vol. 36, 227-234